(19) Europäisches Patentamt
European Patent Office
Office européen des brevets

(11) **EP 2 773 691 B1**

(12) **EUROPÄISCHE PATENTSCHRIFT**

(45) Veröffentlichungstag und Bekanntmachung des Hinweises auf die Patenterteilung:
**15.07.2020 Patentblatt 2020/29**

(21) Anmeldenummer: **12788172.0**

(22) Anmeldetag: **12.11.2012**

(51) Int Cl.:
*C08J 3/12* *(2006.01)*          *A61L 15/60* *(2006.01)*
*A61F 13/53* *(2006.01)*        *C08L 101/14* *(2006.01)*

(86) Internationale Anmeldenummer:
**PCT/EP2012/072360**

(87) Internationale Veröffentlichungsnummer:
**WO 2013/072269 (23.05.2013 Gazette 2013/21)**

(54) **SUPERABSORBIERENDE POLYMERE FÜR HOCHGEFÜLLTE ODER FASERFREIE HYGIENEARTIKEL**

SUPERABSORBENT POLYMERS FOR HIGHLY FILLED AND FIBER-FREE HYGIENE PRODUCTS

POLYMÈRES SUPERABSORBANTS POUR ARTICLES D'HYGIÈNE HAUTEMENT CHARGÉS OU EXEMPTS DE FIBRES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.11.2011 DE 102011086522**

(43) Veröffentlichungstag der Anmeldung:
**10.09.2014 Patentblatt 2014/37**

(73) Patentinhaber: **Evonik Operations GmbH**
**45128 Essen (DE)**

(72) Erfinder:
• HENN, Markus
  45894 Gelsenkirchen (DE)
• WATTEBLED, Laurent
  40589 Düsseldorf (DE)
• HERBE, Peter
  47623 Kevelaer (DE)
• HARREN, Jörg
  52499 Baesweiler (DE)
• LOICK, Christoph
  47918 Tönisvorst (DE)

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
EP-A1- 2 018 877          EP-A1- 2 383 115
WO-A1-02/053198          WO-A1-2006/109844
WO-A1-2008/009580        WO-A1-2009/011717
US-A1- 2005 090 586      US-A1- 2005 288 641
US-A1- 2007 238 806      US-A1- 2010 099 781

EP 2 773 691 B1

**Beschreibung**

Die Erfindung betrifft ein Verfahren zur Herstellung eines absorbierenden Kerns.

Superabsorber sind wasserunlösliche, vernetzte Polymere, die in der Lage sind, unter Quellung und Ausbildung von Hydrogelen große Mengen an wässrigen Flüssigkeiten, insbesondere Körperflüssigkeiten, vorzugsweise Urin oder Blut, aufzunehmen und unter Druck zurückzuhalten. Im Allgemeinen betragen diese Flüssigkeitsaufnahmen das mindestens 10-Fache oder gar das mindestens 100-Fache des Trockengewichts der Superabsorber bzw. der superabsorbierenden Zusammensetzungen an Wasser. Durch diese charakteristischen Eigenschaften finden diese Polymere hauptsächlich Anwendung in den absorbierenden Kernen von Hygieneartikeln wie Babywindeln, Inkontinenzprodukten oder Damenbinden. Einen umfassenden Überblick über Superabsorber bzw. superabsorbierende Zusammensetzungen, ihre Anwendung und ihre Herstellung geben F. L. Buchholz und A. T. Graham (Herausgeber) in "Modern Superabsorbent Polymer Technology", Wiley-VCH, New York, 1998.

Die Herstellung der Superabsorber erfolgt in der Regel durch die radikalische Polymerisation säuregruppentragender, meist teilneutralisierter Monomere in Gegenwart von Vernetzern. Dabei lassen sich durch die Auswahl der Monomerzusammensetzung, der Vernetzer sowie der Polymerisationsbedingungen und der Verarbeitungsbedingungen für das nach der Polymerisation erhaltene Hydrogel Polymere mit unterschiedlichen Absorptionseigenschaften herstellen. Weitere Möglichkeiten bietet die Herstellung von Pfropfpolymerisaten, beispielsweise unter Verwendung chemisch modifizierter Stärke, Cellulose und Polyvinylalkohol nach DE-OS-26 12 846.

Der aktuelle Trend insbesondere im Windelaufbau geht dahin, noch dünnere absorbierende Kerne mit reduziertem Cellulosefaseranteil und erhöhtem Superabsorberanteil herzustellen. Der Vorteil dünnerer Konstruktionen zeigt sich nicht nur in einem verbesserten Tragekomfort, sondern auch in reduzierten Kosten bei Verpackung und Lagerhaltung. Die neuste Generation von absorbierenden Kernen, die beispielsweise in der WO-A-2008/155722, WO-A-2008/155711, WO-A-2008/155710, WO-A-2008/155702, WO-A-2008/155701, WO-A-2008/155699, EP-A-1 225 857, WO-A-01/15647, WO-A-2011/120504, DE-A-10 2009 049 450, WO-A-2008/117109, WO-A-97/11659, EP-A-0 826 349, WO-A-98/37846, WO-A-95/11653, WO-A-95/11651, WO-A-95/11652, WO-A-95/11654, WO-A-2004/071363 oder WO-A-01/89439 beschrieben wird, ist im wesentlichen Cellulose-frei (weswegen entsprechende Windeln auch als *"fluffless diaper"* bezeichnet werden). Die Immobilisierung der Superabsorberpartikel, die in Cellulosehaltigen absorbierenden Kernen durch die Cellulosefasern erfolgt, kann in dieser neuesten Generation absorbierender Kerne dadurch realisiert werden, dass beispielsweise die Superabsorberpartikel mittels thermoplastischer Fasern auf einer Substratoberfläche immobilisiert werden.

Mit dem Trend zu immer dünner werdenden Windelkonstruktionen und dem Wegfall der temporären Flüssigkeitsspeicherungs- und Weiterleitungsfunktion der Cellulosefasern hat sich das Anforderungsprofil an die Superabsorber deutlich verändert. Von entscheidender Bedeutung ist jetzt die Fähigkeit des Hydrogels, das Auslaufen (Leakage) von Urin direkt bei der Miktion zu verhindern. Dies wird durch die Eigenschaft des Superabsorbers/Hydrogels erreicht, die Flüssigkeit während der Quellung effektiv aufzunehmen und in der Gelschicht zu verteilen, bei gleichzeitiger Minimierung der Menge ungebundenen Urins in der Windel. Vorteilhafte Superabsorber führen aufgrund von guten Transporteigenschaften auch zu einer optimalen Ausnutzung des gesamten Hygieneartikels.

Die bisher aus dem Stand der Technik bekannten Superabsorber sind jedoch nur unzureichend für einen Einsatz in der vorstehend beschriebenen neuen Generation von Cellulose-freien Windelkonstruktionen geeignet.

Der vorliegenden Erfindung lag daher die Aufgabe zugrunde, die sich auch dem Stand der Technik ergebenden Nachteile im Zusammenhang mit Superabsorbern zu überwinden.

Insbesondere lag der vorliegenden Erfindung die Aufgabe zugrunde, Superabsorber bereitzustellen, die besonders vorteilhaft in absorbierenden Kernen mit geringem Cellulosefaseranteil, beispielsweise in den in der WO-A-2008/155722, der WO-A-2008/155711, der WO-A-2008/155710, der WO-A-2008/155702, der WO-A-2008/155701 oder der WO-A-2008/155699 beschriebenen, Cellulose-freien Konstruktionen eingesetzt werden können.

Auch lag der vorliegenden Erfindung die Aufgabe zugrunde, ein Selektionsverfahren anzugeben, mittels dessen Superabsorber aus der Vielzahl der derzeit bekannten Superabsobermaterialien ausgewählt werden können, die in zuverlässiger Weise beispielsweise für einen Einsatz in den WO-A-2008/155722, der WO-A-2008/155711, der WO-A-2008/155710, der WO-A-2008/155702, der WO-A-2008/155701 oder der WO-A-2008/155699 beschriebenen, Cellulose-freien Konstruktionen eingesetzt werden können.

Einen Beitrag zur Lösung der eingangs genannten Aufgaben leistet ein teilchenförmiges, absorbierendes Polymermaterial, welches mindestens eine der folgenden Eigenschaften aufweist:

i) einen maximalen $APC_{i \times 10\,sec}$-Wert $\geq 1,6$, bevorzugt $\geq 2,0$ und besonders bevorzugt $\geq 2,4$ für mindestens eine Zahl i ausgewählt aus der Gruppe der ganzen Zahlen von 2 bis 12 (= $APC_{max}$-Wert), wobei vorzugsweise ein $APC_{max}$-Wert von 14,0, besonders bevorzugt von 12,0 und am meisten bevorzugt von 10,0 nicht überschritten wird;
ii) einen Wert für die Summe aller $APC_{i \times 10\,sec}$-Werte $\geq 12$, bevorzugt $\geq 18$ und besonders bevorzugt $\geq 24$ für alle Zahlen i aus der Gruppe der ganzen Zahlen von 2 bis 12 (= $APC_{sum}$); wobei vorzugsweise ein $APC_{sum}$-Wert von

55, besonders bevorzugt von 50 und am meisten bevorzugt von 45 nicht überschritten wird; wobei der APC$_{i \times 10\,sec}$-Wert wie folgt definiert ist:

$$APC_{i \times 10\,sec} = QI_{i \times 10\,sec}\text{-Wert}^2 \times PI_{i \times 10\,sec}\text{-Wert}$$

wobei

- der QI$_{i \times 10\,sec}$-Wert der Wert des gemäß der hierin beschriebenen Testmethode i × 10 Sekunden nach Zugabe der 0,9 gew.-%igen NaCl-Lösung bestimmten Quellindex und

- der PI$_{i \times 10\,sec}$-Wert der Wert des gemäß der hierin beschriebenen Testmethode i × 10 Sekunden nach Zugabe der 0,9 gew.-%igen NaCl-Lösung bestimmten Permeabilitätsindex

sind.

Gemäß einer besonderen Ausgestaltung der teilchenförmigen, absorbierenden Polymermaterialien weisen diese eine gemäß WSP 241.3 (Testverfahren der *"Word Strategie Partners"* EDANA und INDA) bestimmte Zentrifugenretentionskapazität (CRC) von mindestens 22 g/g, noch mehr bevorzugt mindestens 24 g/g, noch mehr bevorzugt mindestens 26 g/g und am meisten bevorzugt mindestens 28 g/g auf.

Gemäß einer weiteren besonderen Ausgestaltung der teilchenförmigen, absorbierenden Polymermaterialien weisen diese eine gemäß WSP 242.3 bestimmte Absorption unter einem Druck von 0,7 psi (AAP) von mindestens 16 g/g, noch mehr bevorzugt mindestens 18 g/g, noch mehr bevorzugt mindestens 20 g/g und am meisten bevorzugt mindestens 22 g/g auf.

Bevorzugte teilchenförmige, absorbierende Polymermaterialien sind Fasern, Schäume oder Teilchen, wobei Fasern und Teilchen bevorzugt und Teilchen besonders bevorzugt sind.

Bevorzugte Polymerfasern sind so dimensioniert, dass sie in oder als Garne für Textilien und auch direkt in Textilien eingearbeitet werden können. Es ist bevorzugt, dass die Polymerfasern eine Länge im Bereich von 1 bis 500 mm, bevorzugt 2 bis 500 mm und besonders bevorzugt 5 bis 100 mm und einen Durchmesser im Bereich von 1 bis 200 Denier, bevorzugt 3 bis 100 Denier und besonders bevorzugt 5 bis 60 Denier besitzen.

Bevorzugte teilchenförmige, absorbierende Polymermaterialien sind so dimensioniert, dass sie eine mittlere Teilchengröße gemäß WSP 220.2 im Bereich von 10 bis 3000 $\mu$m, vorzugsweise 20 bis 2000 $\mu$m und besonders bevorzugt 150 bis 850 $\mu$m aufweisen. Dabei ist es insbesondere bevorzugt, dass der Anteil der Polymerteilchen mit einer Partikelgröße in einem Bereich von 300 bis 600 $\mu$m mindestens 50 Gew.-%, besonders bevorzugt mindestens 65 Gew.-% und am meisten bevorzugt mindestens 80 Gew.-%, bezogen auf das Gesamtgewicht der wasserabsorbierenden Polymerteilchen, beträgt.

Weiterhin ist es bevorzugt, dass die teilchenförmigen, absorbierenden Polymermaterialien auf teilneutralisierter, vernetzter Acrylsäure basieren. In diesem Zusammengang ist es insbesondere bevorzugt, dass die teilchenförmigen absorbierenden Polymermaterialien vernetzte, eine mikroporöse Struktur aufweisende Polyacrylate sind, welche zu mindestens 50 Gew.-%, vorzugsweise zu mindestens 70 Gew.-% und darüber hinaus bevorzugt zu mindestens 90 Gew.-%, jeweils bezogen auf das Gewicht der Polymermaterialien, auf Carboxylatgruppen-tragenden Monomeren bestehen. Es ist weiterhin bevorzugt, dass die teilchenförmigen, absorbierenden Polymermaterialien zu mindestens 50 Gew.-%, vorzugsweise zu mindestens 70 Gew.-%, jeweils bezogen auf das Gewicht der Polymermaterialien, auf polymerisierter Acrylsäure basieren, die vorzugsweise zu mindestens 20 Mol-%, besonders bevorzugt zu mindestens 50 Mol-% und darüber hinaus bevorzugt in einem Bereich von 60 bis 85 Mol-% neutralisiert ist.

Die teilchenförmigen, absorbierenden Polymermaterialien sind vorzugsweise erhältlich durch ein Verfahren beinhaltend die Verfahrensschritte:

i) radikalische Polymerisation einer wässrigen Monomerlösung beinhaltend ein polymerisierbares, monoethylenisch ungesättigtes, eine Säuregruppetragendes Monomer ($\alpha$1) oder ein Salz davon, gegebenenfalls ein mit dem Monomer ($\alpha$1) polymerisierbares, monoethylenisch ungesättigtes Monomer ($\alpha$2), ein Bläh- oder Treibmittel ($\alpha$3) sowie mindestens einen Vernetzer ($\alpha$4) unter Erhalt eines eine mikroporöse Struktur aufweisenden Hydrogels;

ii) gegebenenfalls Zerkleinern des eine mikroporöse Struktur aufweisenden Hydrogels;

iii) Trocknen des gegebenenfalls zerkleinerten, eine mikroporöse Struktur aufweisenden Hydrogels unter Erhalt teilchenförmiger, eine mikroporöse Struktur aufweisender absorbierender Polymermaterialien;

iv) gegebenenfalls Zermahlen und Absieben der so erhaltenen teilchenförmigen, eine mikroporöse Struktur aufweisenden absorbierenden Polymermaterialien;

v) Oberflächennachvernetzung, der so erhaltenen teilchenförmigen, ein mikroporöse Struktur aufweisenden absorbierenden Polymermaterialien mit einem Vernetzer, der mindestens zwei funktionelle Gruppen aufweist, die mit den Säuregruppen auf der Oberfläche der Polymermaterialien zu reagieren vermögen, wobei die Oberfläche der Polymermaterialien vor, während oder nach der Nachvernetzung mit Aluminiumsalzen in Kontakt gebracht wird.

Im Verfahrensschritt i) wird zunächst eine wässrige Monomerlösung beinhaltend ein polymerisierbares, monoethylenisch ungesättigtes, eine Säuregruppetragendes Monomer ($\alpha$1) oder ein Salz davon, gegebenenfalls ein mit dem Monomer ($\alpha$1) polymerisierbares, monoethylenisch ungesättigtes Monomer ($\alpha$2), ein Bläh- oder Treibmittel ($\alpha$3), sowie mindestens einen Vernetzer ($\alpha$4) unter Erhalt eines Polymergels radikalisch polymerisiert. Die monoethylenisch ungesättigten, säuregruppen-tragenden Monomere ($\alpha$1) können teilweise oder vollständig, bevorzugt teilweise neutralisiert sein. Vorzugsweise sind die monoethylenisch ungesättigten, säuregruppen-tragenden Monomere ($\alpha$1) zu mindestens 25 Mol-%, besonders bevorzugt zu mindestens 50 Mol-% und darüber hinaus bevorzugt zu 50-80 Mol-% neutralisiert. In diesem Zusammenhang wird auf DE 195 29 348 A1 verwiesen, deren Offenbarung hiermit als Referenz eingeführt wird. Die Neutralisation kann teilweise oder ganz auch nach der Polymerisation erfolgen. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit einer Kombination aus Carbonaten und/oder Bicarbonaten (die gleichzeitig als Bläh- oder Treibmittel wirken) und Alkalimetallhydroxiden, besonders bevorzugt mit einer Kombination aus Natriumcarbonat und Natriumhydroxid.

Ferner können bei den wasserabsorbierenden Polymergebilden die freien Säuregruppen überwiegen, so dass dieses Polymergebilde einen im sauren Bereich liegenden pH-Wert aufweist. Dieses saure wasserabsorbierende Polymergebilde kann durch ein Polymergebilde mit freien basischen Gruppen, vorzugsweise Amingruppen, das im Vergleich zu dem sauren Polymergebilde basisch ist, mindestens teilweise neutralisiert werden. Diese Polymergebilde werden in der Literatur als *"Mixed-Bed Ion-Exchange Absorbent Polymers"* (MBIEA-Polymere) bezeichnet und sind unter anderem in der WO 99/34843 A1 offenbart. Die Offenbarung der WO 99/34843 A1 wird hiermit als Referenz eingeführt und gilt somit als Teil der Offenbarung. In der Regel stellen MBIEA-Polymere eine Zusammensetzung dar, die zum einen basische Polymergebilde, die in der Lage sind, Anionen auszutauschen, und andererseits ein im Vergleich zu dem basischen Polymergebilde saures Polymergebilde, das in der Lage ist, Kationen auszutauschen, beinhalten. Das basische Polymergebilde weist basische Gruppen auf und wird typischerweise durch die Polymerisation von Monomeren erhalten, die basische Gruppen oder Gruppen tragen, die in basische Gruppen umgewandelt werden können. Bei diesen Monomeren handelt es sich vor allen Dingen um solche, die primäre, sekundäre oder tertiäre Amine oder die entsprechenden Phosphine oder mindestens zwei der vorstehenden funktionellen Gruppen aufweisen. Zu dieser Gruppe von Monomeren gehören insbesondere Ethylenamin, Allylamin, Diallylamin, 4-Aminobuten, Alkyloxycycline, Vinylformamid, 5-Aminopenten, Carbodiimid, Formaldacin, Melamin und dergleichen, sowie deren sekundäre oder tertiäre Aminderivate.

Bevorzugte monoethylenisch ungesättigte, säuregruppen-tragende Monomere ($\alpha$1) sind vorzugsweise diejenigen Verbindungen, die in der WO 2004/037903 A2, die hiermit als Referenz eingeführt wird und somit als Teil der Offenbarung gilt, als ethylenisch ungesättigte, säuregruppenhaltige Monomere ($\alpha$1) genannt werden. Besonders bevorzugte monoethylenisch ungesättigte, säuregruppen-tragende Monomere ($\alpha$1) sind Acrylsäure und Methacrylsäure, wobei Acrylsäure am meisten bevorzugt ist.

Als mit den Monomeren ($\alpha$1) copolymerisierbare, monoethylenisch ungesättigte Monomere ($\alpha$2) können Acrylamide, Methacrylamide oder Vinylamide eingesetzt werden. Weitere bevorzugte Co-Monomere sind insbesondere diejenigen, die in der -tragende Monomere ($\alpha$1) sind vorzugsweise diejenigen Verbindungen, die in der WO 2004/037903 A2 als Co-Monomere ($\alpha$2) genannt werden.

Gemäß einer besonderen Ausführungsform eines Verfahrens zur Herstellung der teilchenförmigen, absorbierenden Polymermaterialien werden als Co-Monomere ($\alpha$2) copolymerisierbare, monoethylenisch ungesättigte Tenside eingesetzt. Diese speziellen "surfactants" enthalten funktionelle Gruppen, die polymerisierbar sind. Bevorzugte chemische Strukturen für diese Tenside sind

$$R^1\text{-}(EO)_n\text{-block-}(PO)_m\text{-}R^2$$

wobei $R^1$ oder $R^2$ Methyl. Ethyl, n-Propyl, iso-Propyl, n-Butyl, iso-Butyl, n-Pentyl, 2-Methyl-Butyl, 2,2-Dimethylpropyl, -OH, Acetyl oder Allyl sind und n = 2 bis 20 und m = 2 bis 20 bedeutet. EO und PO sind respektiv hydrophile und hydrophobe Blöcke, um tensidische Eigenschaften zu generieren. Reaktive AllylGruppen werden für R1 oder R2 bevorzugt, da sie später als Acryl-Gruppe einpolymerisieren und stabiler in der Hydrolyse sind. Als Beispiel eines solchen copolymerisierbaren Tensids sei das kommerzielle von der BASF AG eingesetzte Produkt Pluriol A 23 R genannt.

Als Bläh- oder Treibmittel ($\alpha$3) kommen insbesondere kommen alle dem Fachmann bekannten Hilfsstoffe in Betracht, die bei der Polymerisation die Bildung von Hohlräumen, beispielsweise von Poren, und mithin die Bildung einer mikroporösen Struktur im Polymergel begünstigen. Als Treibmittel kommen dabei insbesondere diejenigen Treibmittel in Betracht, die etwa in der EP-A-0-644 207 als bevorzugte Treibmittel offenbart werden. In Betracht kommen hierbei insbesondere Treibmittel auf der Basis von Carbonaten. Beim Erhitzen setzt das Treibmittel Kohlendioxid frei, welches in der carbonathaltigen Monomerlösung gelöst oder dispergiert ist. Das Treibmittel kann jedes Carbonat oder Bicarbonat enthaltende Salz oder gemischtes Salz sein und kann Kohlendioxid als ein Gas oder Feststoff Natriumcarbonat, Kaliumcarbonat, Ammoniumcarbonat, Magnesiumcarbonat oder Magnesium(hydroxy)carbonate, Calciumcarbonat, Bariumcarbonat, Bicarbonate und deren Hydrate oder andere Kationen, sowie natürlich vorkommende Carbonate wie Dolomit und dessen Mischungen umfassen. Bevorzugte Carbonattreibmittel sind $MgCO_3$, $(NH4)_2CO_3$ $Na_2CO_3$ und deren Mischungen. In diesem Zusammenhang ist es weiterhin bevorzugt, dass etwa 0,05 bis etwa 5,0 Gew.-% Treibmittel, bezogen auf das Gewicht des gegebenenfalls teilneutralisierten Monomers ($\alpha$1), besonders bevorzugt bezogen auf die gegebenenfalls teilneutralisierte Acrylsäure, zugefügt werden. Am meisten bevorzugt ist es, etwa 0,2 Gew.-% bis etwa 3,0 Gew.-% Treibmittel zuzufügen. Die Treibmittel können dabei auch Eingekapselt werden, wie dies beispielsweise in der US 7,163,966 beschrieben ist.

Neben oder anstelle der vorstehend beschriebenen Treibmittel können der Monomerlösung zum Zwecke der Ausbildung von Hohlräumen in Polymermaterial auch Hohlkörper mit einer Hülle aus einem anorganischen oder organischen Material zugesetzt werden, wie dies in der WO-A-2010/115671 beschrieben ist. Als Hohlkörper mit einer Hülle aus einem organischen Material werden vorzugsweise Hohlkörper ausgewählt aus der folgenden Gruppe verstanden:

- Hohlkörper, welche eine Hülle aus einem polymeren, thermoplastischen Material aufweisen;
- Hohlkörper, welche eine Hülle aus einem polymeren, nicht-thermoplastischen Material aufweisen.

Generell können als Hohlkörper

- auf thermoplastischen oder nicht-thermoplastischen Polymeren basierende, gasgefüllte Mikroballons ("*gas filled microballons*"),
- polyelektrolytische, mehrschichtige Kapseln ("*polyelectrolyte multilayer capsules*"),
- auf thermoplastischen oder nicht-thermoplastischen Polymeren basierende, sogenannte Hohlsphären ("*hollow spheres*"),
- auf thermoplastischen Polymeren basierende Microsphere-Partikel, wie sie beispielsweise unter der Handelsbezeichnung "EXPANCEL®" erhältlich sind, oder
- Hohlkörper mit einer Hülle aus polykristallinem Aluminiumoxideingesetzt werden.

Als Vernetzer ($\alpha$4) werden vorzugsweise ebenfalls diejenigen Verbindungen eingesetzt, die in der WO 2004/037903 A2 als Vernetzer ($\alpha$3) Ist diese Aufzahlung auf die WO2004037903 bezogen? Ansonsten müsste es ($\alpha$4) heißen! genannt werden. Unter diesen Vernetzern sind wasserlösliche Vernetzer besonders bevorzugt. Am meisten bevorzugt sind dabei N,N'-Methylenbisacrylamid, Polyethylenglykoldi-(meth)acrylate, Triallylmethylammoniumchlorid, Tetraallylammoniumchlorid sowie mit 9 Mol Ethylenoxid pro Mol Acrylsäure hergestelltes Allylnonaethylenglykolacrylat.

Neben den Monomeren ($\alpha$1) und gegebenenfalls ($\alpha$2), den Bläh- oder Treibmitteln ($\alpha$3) sowie dem mindestens einen Vernetzer ($\alpha$4) kann die Monomerlösung auch wasserlösliche Polymere ($\alpha$5) beinhalten. Bevorzugte wasserlösliche Polymere umfassend teil- oder vollverseiften Polyvinylalkohol, Polyvinylpyrrolidon, Stärke oder Stärkederivate, Polyglykole oder Polyacrylsäure. Das Molekulargewicht dieser Polymere ist unkritisch, solange sie wasserlöslich sind. Bevorzugte wasserlösliche Polymere sind Stärke oder Stärkederivate oder Polyvinylalkohol.

Die wasserlöslichen Polymere, vorzugsweise synthetische wie Polyvinylalkohol, können nicht nur als Pfropfgrundlage für die zu polymerisierenden Monomeren dienen. Denkbar ist auch, diese wasserlöslichen Polymere erst nach der Polymerisation mit dem Polymergel oder dem bereits getrockneten, wasserabsorbierenden Polymergel zu vermischen.

Weiterhin kann die Monomerlösung auch Hilfsmittel ($\alpha$6) enthalten, wobei zu diesen Hilfsmitteln insbesondere die für die Polymerisation gegebenenfalls erforderlichen Initiatoren oder Komplexbildner, wie beispielsweise EDTA, gehören.

Als Lösungsmittel für die Monomerlösung kommen Wasser, organische Lösungsmittel oder Gemische aus Wasser und organischen Lösungsmitteln in Betracht, wobei die Wahl des Lösungsmittels insbesondere auch von der Art und Weise der Polymerisation abhängt.

Die relative Menge an Monomeren ($\alpha$1) und ($\alpha$2), an Bläh- oder Treibmitteln ($\alpha$3) sowie an Vernetzern ($\alpha$4) und wasserlöslichen Polymeren ($\alpha$5) und Hilfsmittel ($\alpha$6) in der Monomerlösung wird vorzugsweise so gewählt, dass das im Verfahrensschritt iii) nach dem Trocknen erhaltene teilchenförmige, eine mikroporöse Struktur aufweisende absorbierende Polymermaterial

- zu 20 bis 99,999 Gew.-%, bevorzugt zu 55 bis 98,99 Gew.-% und besonders bevorzugt zu 70 bis 98,79 Gew.-% auf den Monomeren ($\alpha$1),
- zu 0 bis 80 Gew.-%, vorzugsweise zu 0 bis 44,99 Gew.-% und besonders bevorzugt zu 0,1 bis 44,89 Gew.-% auf den Monomeren (α2),

- zu 0 bis 5 Gew.-%, vorzugsweise zu 0,001 bis 3 Gew.-% und besonders bevorzugt zu 0,01 bis 2,5 Gew.-% auf den Vernetzern (α4),
- zu 0 bis 30 Gew.-%, vorzugsweise zu 0 bis 5 Gew.-% und besonders bevorzugt zu 0,1 bis 5 Gew.-% auf den wasserlöslichen Polymeren (α5),
- zu 0 bis 20 Gew.-%, vorzugsweise zu 0 bis 10 Gew.-% und besonders bevorzugt zu 0,1 bis 8 Gew.-% auf den Hilfsmitteln (α6), und
- zu 0,5 bis 25 Gew.-%, vorzugsweise zu 1 bis 10 Gew.-% und besonders bevorzugt zu 3 bis 7 Gew.-% auf Wasser (a7)

basiert, wobei die Summe der Gewichtsmengen (α1) bis (α7) 100 Gew.-% beträgt. Optimale Werte für die Konzentration insbesondere der Monomere, der Treib- oder Blähmittel, Vernetzer und wasserlöslichen Polymere in der Monomerlösung können durch einfache Vorversuche ermittelt oder aber auch dem Stand der Technik, insbesondere den Druckschriften US 4,286,082, DE-A-27 06 135, US 4,076,663, DE-A-35 03 458, DE 40 20 780 Cl, DE-A-42 44 548, DE-A-43 33 056 und DE-A-44 18 818 entnommen werden. Zur radikalischen Polymerisation der Monomerlösung können grundsätzlich alle dem Fachmann bekannten Polymerisationsverfahren in Betracht kommen. Beispielsweise sind in diesem Zusammenhang Lösungspolymerisation, die vorzugsweise in Knetreaktoren wie Extrudern oder kontinuierlich auf einem Polymerisationsband erfolgt, Spraypolymerisation, inverse Emulsionspolymerisation und inverse Suspensionspolymerisation zu nennen.

Bevorzugt wird die Lösungspolymerisation in Wasser als Lösungsmittel durchgeführt. Die Lösungspolymerisation kann kontinuierlich oder diskontinuierlich erfolgen. Aus dem Stand der Technik ist ein breites Spektrum von Variationsmöglichkeiten hinsichtlich Reaktionsverhältnisse wie Temperaturen, Art und Menge der Initiatoren als auch der Reaktionslösung zu entnehmen. Typische Verfahren sind in den folgenden Patentschriften beschrieben: US 4,286,082, DE-A-27 06 135 A1, US 4,076,663, DE-A-35 03 458, DE 40 20 780 C1, DE-A-42 44 548, DE-A-43 33 056, DE-A-44 18 818. Die Offenbarungen werden hiermit als Referenz eingeführt und gelten somit als Teil der Offenbarung.

Die Polymerisation wird wie allgemein üblich durch einen Initiator ausgelöst. Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Auch eine Initiierung der Polymerisation durch Einwirkung von Elektronenstrahlen auf die polymerisierbare, wässrige Mischung ist möglich. Die Polymerisation kann allerdings auch in Abwesenheit von Initiatoren der oben genannten Art durch Einwirkung energiereicher Strahlung in Gegenwart von Photoinitiatoren ausgelöst werden. Polymerisationsinitiatoren können in der Monomerlösung gelöst oder dispergiert enthalten sein. Als Initiatoren kommen sämtliche dem Fachmann bekannte in Radikale zerfallende Verbindungen in Betracht. Hierunter fallen insbesondere diejenigen Initiatoren, die bereits in der WO-A-2004/037903 als mögliche Initiatoren genannt werden. Besonders bevorzugt wird zur Herstellung der wasserabsorbierenden Polymergebilde ein Redoxsystem bestehend aus Wasserstoffperoxid, Natriumperoxodisulfat und Ascorbinsäure eingesetzt.

Auch die inverse Suspensions- und Emulsionspolymerisation kann zur Herstellung der teilchenförmigen absorbierenden Polymermaterialien angewendet werden. Gemäß diesen Prozessen wird eine wässrige, teilneutralisierte Lösung die Monomeren (α1) und die Treib- oder Blähmittel (α3), sowie gegebenenfalls beinhaltend die weiteren Monomere (α2), die wasserlöslichen Polymere (α5) und Hilfsmittel (α6), mit Hilfe von Schutzkolloiden und/oder Emulgatoren in einem hydrophoben, organischen Lösungsmittel dispergiert und durch Radikalinitiatoren die Polymerisation gestartet. Die Vernetzer (α4) sind entweder in der Monomerlösung gelöst und werden mit dieser zusammen dosiert oder aber separat und gegebenenfalls während der Polymerisation zugefügt. Gegebenenfalls erfolgt die Zugabe eines wasserlöslichen Polymeren (α5) als Pfropfgrundlage über die Monomerlösung oder durch direkte Vorlage in die Ölphase. Anschließend wird das Wasser azeotrop aus dem Gemisch entfernt und das Polymerisat abfiltriert.

Weiterhin kann sowohl bei der Lösungspolymerisation als auch bei der inversen Suspensions- und Emulsionspolymerisation die Vernetzung durch Einpolymerisation des in der Monomerlösung gelösten polyfunktionellen Vernetzers (α4) und/oder durch Reaktion geeigneter Vernetzer mit funktionellen Gruppen des Polymeren während der Polymerisationsschritte erfolgen. Die Verfahren sind beispielsweise in den Veröffentlichungen US 4,340,706, DE-A-37 13 601, DE-A-28 40 010 und WO-A-96/05234 beschrieben, deren entsprechende Offenbarung hiermit als Referenz eingeführt wird.

Im Verfahrensschritt ii) wird das im Verfahrensschritt i) erhaltene, eine mikroporöse Struktur aufweisende Hydrogel gegebenenfalls zerkleinert, wobei dieses Zerkleinern insbesondere dann erfolgt, wenn die Polymerisation mittels einer Lösungspolymerisation durchgeführt wird. Das Zerkleinern kann durch dem Fachmann bekannte Zerkleinerungsvorrichtungen, wie etwa einem Fleischwolf, erfolgen.

Im Verfahrensschritt iii) wird das gegebenenfalls zuvor zerkleinerte, eine mikroporöse Struktur aufweisende Hydrogel getrocknet. Die Trocknung des Hydrogels erfolgt vorzugsweise in geeigneten Trocknern oder Öfen. Beispielhaft seien Drehrohröfen, Wirbelbetttrockner, Tellertrockner, Paddeltrockner oder Infrarottrockner genannt. Weiterhin ist es bevorzugt, dass die Trocknung des Hydrogels im Verfahrensschritt iii) bis zu einem Wassergehalt von 0,5 bis 25 Gew.-%, vorzugsweise von 1 bis 10 Gew.-% erfolgt, wobei die Trocknungstemperaturen üblicherweise in einem Bereich von 100 bis 200°C liegen.

Im Verfahrensschritt iv) können die im Verfahrensschritt iii) erhaltenen, eine mikroporöse Struktur aufweisenden teilchenförmigen, absorbierenden Polymermaterialien insbesondere dann, wenn sie durch Lösungspolymerisation erhalten wurden, noch zermahlen und auf die eingangs genannte Wunschkorngröße abgesiebt werden. Das Zermahlen der getrockneten, eine mikroporöse Struktur aufweisenden absorbierenden Polymermaterialien erfolgt vorzugsweise in geeigneten, mechanischen Zerkleinerungsvorrichtungen, wie etwa einer Kugelmühle, während das Absieben beispielsweise durch Verwendung von Sieben mit geeigneter Maschenweite erfolgen kann.

Im Verfahrensschritt v) werden die gegebenenfalls zermahlenen und abgesiebten teilchenförmigen, eine mikroporöse Struktur aufweisenden absorbierenden Polymermaterialien mit einem Vernetzer, der mindestens zwei funktionelle Gruppen aufweist, die mit den Säuregruppen auf der Oberfläche der Polymermaterialien zu reagieren vermögen, oberflächennachvernetzt, wobei die Oberfläche der Polymermaterialien vor, während oder nach der Nachvernetzung mit Aluminiumsalzen, vorzugsweise mit Aluminiumlactat und/oder Aluminiumsulfat, in Kontakt gebracht wird.

Zur Oberflächennachvernetzung werden die getrockneten und gegebenenfalls zermahlenen und abgesiebten teilchenförmigen, eine mikroporöse Struktur aufweisenden, absorbierenden Polymermaterialien aus den Verfahrensschritten iii) oder iv) oder aber das noch nicht getrocknete, jedoch vorzugsweise bereits zerkleinerte, eine mikroporöse Struktur aufweisende Hydrogel aus dem Verfahrensschritt ii) mit einem vorzugsweise organischen, chemischen Oberflächennachvernetzer in Kontakt gebracht. Dabei wird der Nachvernetzer insbesondere dann, wenn er unter den Nachvernetzungsbedingungen nicht flüssig ist, vorzugsweise in Form eines Fluids umfassend den Nachvernetzer sowie ein Lösungsmittel mit den teilchenförmigen absorbierenden Polymermaterial bzw. dem Polymergel in Kontakt gebracht. Als Lösungsmittel werden dabei vorzugsweise Wasser, mit Wasser mischbare organische Lösungsmittel wie etwa Methanol, Ethanol, 1-Propanol, 2-Propanol oder 1-Butanol oder Mischungen aus mindestens zwei dieser Lösungsmittel eingesetzt, wobei Wasser als Lösungsmittel am meisten bevorzugt ist. Weiterhin ist es bevorzugt, dass der Nachvernetzer in dem Fluid in einer Menge in einem Bereich von 5 bis 75 Gew.-%, besonders bevorzugt 10 bis 50 Gew.-% und am meisten bevorzugt 15 bis 40 Gew.-%, bezogen auf das Gesamtgewicht des Fluids, enthalten ist.

Das in Kontakt bringen des teilchenförmigen, eine mikroporöse Struktur aufweisenden absorbierenden Polymermaterials bzw. des gegebenenfalls zerkleinerten, eine mikroporöse Struktur aufweisenden Hydrogels mit dem Fluid beinhaltend den Nachvernetzer erfolgt vorzugsweise durch gutes Vermischen des Fluids mit dem Polymermaterial bzw. dem Hydrogel.

Geeignete Mischaggregate zum Aufbringen des Fluids sind z. B. der Patterson Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Polymermaterial mittels rotierender Messer in schneller Frequenz gemischt wird (Schugi-Mischer).

Das teilchenförmige, eine mikroporöse Struktur aufweisende absorbierende Polymermaterial bzw. das Hydrogel wird bei der Nachvernetzung vorzugsweise mit höchstens 20 Gew. -%, besonders bevorzugt mit höchstens 15 Gew.-%, darüber hinaus bevorzugt mit höchstens 10 Gew. -%, darüber hinaus noch mehr bevorzugt mit höchstens 5 Gew.-% an Lösungsmittel, vorzugsweise Wasser, in Kontakt gebracht.

Bei Polymermaterialien in der Form von vorzugsweise kugelförmigen Teilchen ist es weiterhin bevorzugt, dass das in Kontakt bringen derart erfolgt, dass lediglich der Aussenbereich, nicht jedoch der innere Bereich der teilchenförmigen Polymermaterialien mit dem Fluid und somit dem Nachvernetzer in Kontakt gebracht werden.

Als Nachvernetzer werden vorzugsweise Verbindungen verstanden, die mindestens zwei funktionelle Gruppen aufweisen, die mit den Säuregruppen auf der Oberfläche des Polymermaterials in einer Kondensationsreaktion (=Kondensationsvernetzer), in einer Additionsreaktion oder in einer Ringöffnungsreaktion reagieren können. Als Nachvernetzer sind diejenigen bevorzugt, die in WO-A-2004/037903 als Vernetzer der Vernetzerklassen II genannt wurden.

Unter diesen Verbindungen sind als Nachvernetzer besonders bevorzugt Kondensationsvernetzer wie beispielsweise Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylensorbitanfettsäureester, Trimethylolpropan, Pentaerytrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on sowie 1,3-Dioxolan-2-on.

Nachdem das teilchenförmige, eine mikroporöse Struktur aufweisende absorbierende Polymermaterial bzw. die eine mikroporöse Struktur aufweisenden Hydrogele mit dem Nachvernetzer bzw. mit dem Fluid beinhaltend den Nachvernetzer in Kontakt gebracht wurden, werden sie auf eine Temperatur im Bereich von 50 bis 300°C, vorzugsweise 75 bis 275°C und besonders bevorzugt 150 bis 250°C erhitzt, so dass, vorzugsweise wodurch, der Außenbereich der Teilchen des Polymermaterials im Vergleich zum Innenbereich stärker vernetzt (=Nachvernetzung) und, sofern ein Hydrogel eingesetzt werden, diese zugleich auch getrocknet werden. Die Zeitdauer der Wärmebehandlung wird durch die Gefahr, dass das gewünschte Eigenschaftsprofil des Polymermaterials infolge von Hitzeeinwirkung zerstört wird, begrenzt.

Vor, während oder nach der Nachvernetzung wird die Oberfläche der Polymermaterialien bzw. des eine mikroporöse Struktur aufweisenden Hydrogels mit Aluminiumsalzen, vorzugsweise mit Aluminiumlactat, in Kontakt gebracht. Besonders bevorzugt ist es, dass die Behandlung mit den Aluminiumsalzen zeitgleich mit der Oberflächennachvernetzung durchgeführt wird, indem eine vorzugsweise wässrige Lösung beinhaltend den Nachvernetzter sowie das bzw. die Aluminiumsalze, vorzugsweise Aluminiumlactat, , mit dem teilchenförmigen, eine mikroporöse Struktur aufweisenden absorbierenden Polymermaterial bzw. mit dem eine mikroporöse Struktur aufweisenden Hydrogel in Kontakt gebracht und dann erhitzt wird.

Dabei ist es bevorzugt, dass die Aluminiumsalze in einer Menge in einem Bereich von 0,01 bis 30 Gew.-%, besonders bevorzugt in einer Menge in einem Bereich von 0,1 bis 20 Gew.-% und darüber hinaus bevorzugt in einer Menge in einem Bereich von 0,3 bis 5 Gew.-% (sofern als Hydrat vorliegend, wasserfrei berechnet), jeweils bezogen auf das Gewicht des teilchenförmigen, eine mikroporöse Struktur aufweisenden absorbierenden Polymermaterial bzw. des eine mikroporöse Struktur aufweisenden Hydrogels, mit dem Polymermaterial bzw. dem Hydrogel in Kontakt gebracht wird.

Bevorzugte Aluminiumsalze sind insbesondere $AlCl_3$ x $6H_2O$, $NaAl(SO_4)_2$ x 12 $H_2O$, $KAl(SO_4)_2$ x 12 $H_2O$ oder $Al_2(SO_4)_3$ x 14-18 $H_2O$, Aluminiumlactat oder aber wasserunlösliche Aluminiumverbindungen, wie etwa Aluminumoxide, beispielsweise $Al_2O_3$, oder Aluminate. Besonders bevorzugt werden Aluminiumlactat, Aluminiumsulfat oder Michungen aus Aluminiumlactat und Aluminiumsulfat eingesetzt.

Einen Beitrag zur Lösung der eingangs genannten Aufgaben leistet auch ein absorbierender Kern, beinhaltend eine obere Substratschicht, eine untere Substratschicht und eine zwischen der oberen und der unteren Substratschicht angeordnete Absorptionsschicht,
wobei die Absorptionsschicht ein erfindungsgemäß selektiertes teilchenförmiges, absorbierendes Polymermaterial und weniger als 0,1 g, besonders bevorzugt weniger als 0,05 g und am meisten bevorzugt weniger als 0,01 g Cellulosefasern pro Gramm des teilchenförmigen, absorbierenden Polymermaterials beinhaltet.

Unter einem *"absorbierenden Kern"* wird dabei erfindungsgemäß vorzugsweise eine Konstruktion verstanden, die zwischen der der Körperseite des Trägers abgewandten, für wässrige Flüssigkeiten impermeablen oberen Lage und der der Körperseite des Trägers zugewandten, für wässrige Flüssigkeiten permeablen unteren Lage eines Absorptionsartikels, wie etwa einer Windel, angeordnet sein kann und dessen Funktion in erster Linie darin besteht, die von dem Absorptionsartikel aufgenommenen Flüssigkeiten, wie beispielsweise Blut oder Urin, zu absorbieren und zu speichern. Der absorbierende Kern selbst umfasst vorzugsweise kein Aufnahmesystem, keine obere Lage und keine untere Lage des Absorptionsartikels.

Am meisten bevorzugt ist es jedoch, wenn der absorbierende Kern im Wesentlich frei von Cellulosefasern ist. Die Formulierung *"im Wesentlichen frei von Cellulosefasern"* wird hierin verwendet, um einen Artikel, wie einen absorbierenden Kern, zu beschreiben, der zu weniger als 10 Gew.-% Cellulosefasern, zu weniger als 5 Gew.-% Cellulosefasern, zu weniger als 1 Gew.-% Cellulosefasern, keine Cellulosefasern oder nicht mehr als eine unerhebliche Menge an Cellulosefasern beinhaltet. Solche Kerne sind insbesondere in WO-A-2008/155722, WO-A-2008/155711, WO-A-2008/155710, WO-A-2008/155702, WO-A-2008/155701, WO-A-2008/155699, EP-A-1 225 857, WO-A-01/15647, WO-A-2011/120504, DE-A-10 2009 049 450, WO-A-2008/117109, WO-A-97/11659, EP-A-0 826 349, WO-A-98/37846, WO-A-95/11653, WO-A-95/11651, WO-A-95/11652, WO-A-95/11654, WO-A-2004/071363 oder WO-A-01/89439 beschrieben.

Gemäß einer bevorzugten Ausführungsform des absorbierenden Kerns beinhaltet der absorbierende Kern eine Vielzahl von das teilchenförmige absorbierende Polymermaterial beinhaltende Kompartimente sowie ein thermoplastisches Material. In diesem Zusammenhang erfindungsgemäß besonders bevorzugte absorbierende Kerne sind solche Konstruktionen, die in der WO-A-2008/155722, der WO-A-2008/155711, der WO-A-2008/155710, der WO-A-2008/155702, der WO-A-2008/155701 oder der WO-A-2008/155699 als *"absorbent core"* bezeichnet und beschrieben werden. Demnach ist es erfindungsgemäß besonders bevorzugt, dass der absorbierende Kern eine Vielzahl von das teilchenförmige absorbierende Polymermaterial beinhaltende Kompartimente sowie Schichten eines thermoplastischen Materials beinhaltet, wobei die Kompartimente jeweils von der oberen oder der unteren Substratschicht sowie von einer Schicht des thermoplastischen Material begrenzt werden. Realisiert werden kann eine solche Konstruktion gemäß der Lehre der vorstehend zitierten Patentanmeldungen dadurch, dass das teilchenförmige absorbierende Polymermaterial auf den jeweiligen Substraten (untere bzw. obere Substratschicht) in Clustern von Teilchen aufgetragen wird, um ein Rastermuster zu bilden, das Inselbereiche und Verbindungsbereiche zwischen den Inselbereichen bildet. Dabei sind *"Inselbereiche"* Bereiche, in denen das thermoplastische Material die untere bzw. obere Substratschicht nicht direkt berührt. *"Verbindungsbereiche"* sind Bereiche, in denen das thermoplastische Material die untere bzw. obere Substratschicht direkt berührt. Die Verbindungsbereiche in dem Rastermuster enthalten wenig oder kein teilchenförmiges absorbierendes Polymermaterial. Die Inselbereiche und Verbindungsbereiche können in einer Vielfalt von Formen vorliegen, einschließlich, jedoch nicht beschränkt auf kreisförmig, oval, quadratisch, rechteckig, dreieckig und dergleichen.

Als ein Beispiel eines absorbierenden Kerns sei ein Kern genannt, der eine erste Teilschicht und eine an die erste Teilschicht angrenzende zweite Teilschicht beinhaltet, wobei

- die erste Teilschicht die obere Substratschicht sowie das teilchenförmige absorbierende Polymermaterial beinhaltende Kompartimente beinhaltet, die von der oberen Substratschicht und einer ersten Schicht eines thermoplastischen Materials begrenzt sind, und

- die zweite Teilschicht die untere Substratschicht sowie das teilchenförmige absorbierende Polymermaterial beinhaltende Kompartimente beinhaltet, die von der unteren Substratschicht und einer zweiten Schicht eines thermoplastischen Materials begrenzt sind,

wobei die erste Schicht des thermoplastischen Materials an die zweite Schicht des thermoplastischen Materials angrenzt. Diese Konstruktion entspricht derjenigen Konstruktion, die beispielsweise in den Figuren 7A und 7B der WO-A-2008/155722 gezeigt ist.

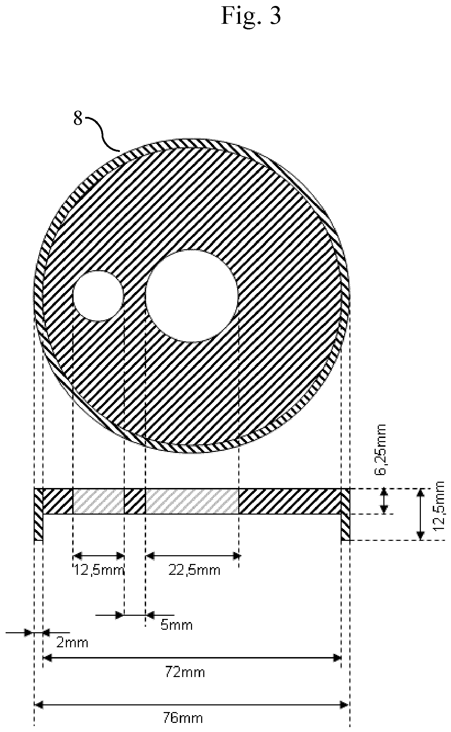

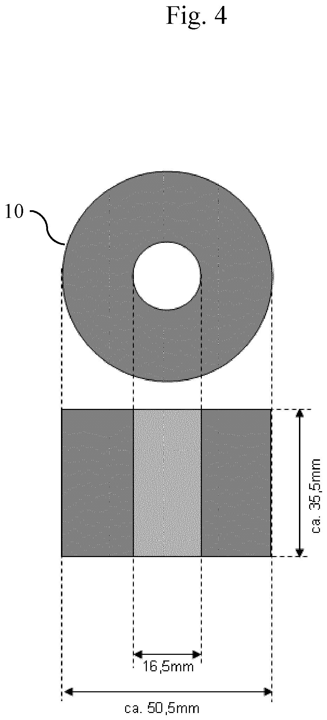

Das thermoplastische Material in der bevorzugten Konstruktion des absorbierenden Kerns dient in erster Linie dazu, das teilchenförmige absorbierende Polymermaterial zu bedecken und mindestens teilweise auf der oberen bzw. unteren Substratschicht zu immobilisieren. In einer Ausführungsform der vorliegenden Erfindung kann das thermoplastische Material im Wesentlichen gleichmäßig innerhalb des teilchenförmigen absorbierenden Polymermaterials zwischen den Polymeren angeordnet sein. Jedoch kann in einer bestimmten Ausführungsform das thermoplastische Material als Faserschicht bereitgestellt sein, die mindestens teilweise in Kontakt mit dem teilchenförmigen absorbierenden Polymermaterial und teilweise in Kontakt mit der oberen bzw. unteren Substratschicht steht, wie dies etwa in den Figuren 3 und 4 der WO-A-2008/155722 gezeigt ist. In diesem Falle kann die Schicht des thermoplastischen Materials beispielsweise durch das Verschmelzen eines faserförmigen thermoplastischen Materials erhältlich sein.

Als Materialien für die obere und untere Substratschicht sowie für das thermoplastische Material sind dabei diejenigen Materialien bevorzugt, die beispielsweise in der WO-A-2008/155722 als für diese Komponenten bevorzugte Materialien beschrieben werden.

Die Art und Weise der Herstellung derartiger absorbierender Kerne entspricht vorzugsweise derjenigen Art und Weise, die beispielsweise in der WO-A-2008/155699 beschrieben ist. Demnach umfasst das Verfahren die Verfahrensschritte:

- das Aufbringen des teilchenförmigen absorbierenden Polymermaterials auf der unteren Substratschicht in einem ersten Muster, um eine erste Absorptionsschicht zu bilden, so dass das teilchenförmige absorbierende Polymermaterial diskontinuierlich auf der unteren Substratschicht verteilt wird;

- das Aufbringen des teilchenförmigem absorbierenden Polymermaterials auf die obere Substratschicht in einem zweiten Muster, um eine zweite Absorptionsschicht zu bilden, so dass das teilchenförmige absorbierende Polymermaterial diskontinuierlich auf der oberen Substratschicht verteilt wird;

- das Aufbringen der Schicht des thermoplastischen Materials auf dem teilchenförmigen absorbierenden Polymermaterial und der unteren und oberen Substratschicht, um das teilchenförmige absorbierende Polymermaterial auf der unteren und oberen Substratschicht abzudecken; und

- das Miteinanderkombinieren der ersten und zweiten Absorptionsschichten, so dass mindestens ein Abschnitt des thermoplastischen Materials der ersten Absorptionsschicht mindestens einen Abschnitt des thermoplastischen Materials der zweiten Absorptionsschicht berührt.

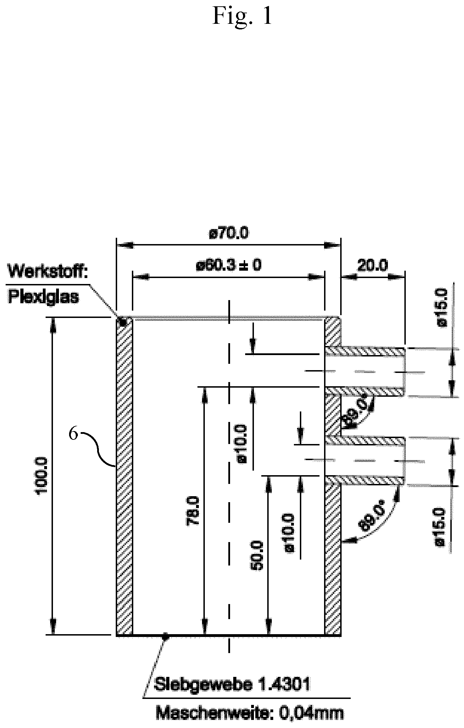

Einen Beitrag zur Lösung der eingangs genannten Aufgaben leistet auch ein Hygieneartikel, beinhaltend einen absorbierenden Kern, wobei es sich bei diesem Hygieneartikel vorzugsweise um eine Windel handelt. Eine Windel beinhaltet üblicherweise eine für Flüssigkeiten permeable untere Lage (die, wenn die Windel getragen wird, der Körperseite des Windelträgers zugewandt ist), eine für Flüssigkeiten im Wesentlichen impermeable obere Lage (die, wenn die Windel getragen wird, der Körperseite des Windelträgers abgewandt ist) sowie den zwischen der unteren Lage und der oberen Lage angeordneten absorbierenden Kern. Der Hygieneartikel kann neben diesen drei Komponenten auch weitere Komponenten beinhalten, wie etwa ein wiederverschließbares Befestigungssystem, wie es beispielsweise in der WO-A-2008155699 beschrieben ist, oder eine weitere Aufnahmeschicht, die zwischen der für Flüssigkeiten permeablen unteren Lage und dem absorbierenden Kern lokalisiert ist, wie sie beispielsweise in der WO-A-2008/155722 beschrieben ist. Eine bevorzugte Windelkonstruktion ist beispielsweise in der Figur 1 der WO-A-2008/155722 gezeigt.

Gegenstand der Erfindung ist ein Verfahren zur Herstellung eines absorbierender Kerns, welcher eine obere Substratschicht, eine untere Substratschicht und eine zwischen der oberen und der unteren Substratschicht angeordnete Absorptionsschicht beinhaltet, wobei die Absorptionsschicht ein teilchenförmiges absorbierendes Polymermaterial und weniger als 0,1 g Cellulosefasern pro Gramm des teilchenförmigen absorbierenden Polymermaterials umfasst, beinhaltend die Verfahrensschritte:

A) Bereitstellen eines teilchenförmigen absorbierenden Polymermaterials;

B) Einarbeiten des teilchenförmigen absorbierenden Materials in einen absorbierenden Kern;

mit den Merkmalen des Anspruch 1.

Beispielsweise umfasst dabei das Einarbeiten des teilchenförmigen Materials in den absorbierenden Kern die Verfahrensschritte:

B1) das Aufbringen des teilchenförmigen absorbierenden Polymermaterials auf der unteren Substratschicht in einem ersten Muster, um eine erste Absorptionsschicht zu bilden, so dass das teilchenförmige absorbierende Polymermaterial diskontinuierlich auf der unteren Substratschicht verteilt wird;

B2) das Aufbringen des teilchenförmigem absorbierenden Polymermaterials auf die obere Substratschicht in einem zweiten Muster, um eine zweite Absorptionsschicht zu bilden, so dass das teilchenförmige absorbierende Polymermaterial diskontinuierlich auf der oberen Substratschicht verteilt wird;

B3) das Aufbringen der Schicht des thermoplastischen Materials auf dem teilchenförmigen absorbierenden Polymermaterial und der unteren und oberen Substratschicht, um das teilchenförmige absorbierende Polymermaterial auf der unteren und oberen Substratschicht abzudecken; und

B4) das Miteinanderkombinieren der ersten und zweiten Absorptionsschichten, so dass mindestens ein Abschnitt des thermoplastischen Materials der ersten Absorptionsschicht mindestens einen Abschnitt des thermoplastischen Materials der zweiten Absorptionsschicht berührt.

Ein Verfahren zur Herstellung eines absorbierender Kerns, welcher eine obere Substratschicht, eine untere Substratschicht und eine zwischen der oberen und der unteren Substratschicht angeordnete Absorptionsschicht beinhaltet, wobei die Absorptionsschicht ein teilchenförmiges absorbierendes Polymermaterial und weniger als 0,1 g Cellulosefasern pro Gramm des teilchenförmigen absorbierenden Polymermaterials umfasst,
umfasst gemäß der Erfindung die Verfahrensschritte:

a) Selektion eines geeigneten teilchenförmigen absorbierenden Polymermaterials auf der Basis der Ergebnisse der Bestimmung des Produktes aus (Quellindex)$^2$ und (Permeabilitätsindex) (= APC-Wert) für mindestens einen Zeitpunkt $t_i$ nach Zugabe einer Flüssigkeit zu dem teilchenförmigen absorbierenden Polymergebilde;

b) Einarbeiten eines auf diese Weise selektierten teilchenförmigen absorbierenden Materials in den absorbierenden Kern,

wobei als absorbierende Kerne diejenigen Konstruktionen bevorzugt sind, die bereits eingangs im Zusammenhang mit dem absorbierenden Kern als bevorzugte Konstruktionen beschrieben worden sind.
Gemäß des erfindungsgemäßen Verfahrens wird im Verfahrensschritt a) das Produkt aus Quellindex$^2$ und Permeabilitätsindex (= APC-Wert) für mindestens 11 verschiedene Zeitpunkte $t_i$ bestimmt und die Selektion des teilchenförmigen absorbierenden Polymermaterials erfolgt auf der Basis der Summe aller Erhaltenen APC-Werte.
Einen Beitrag zur Lösung der eingangs genannten Aufgaben leistet auch ein absorbierender Kern, welcher durch die vorstehend beschriebenen Verfahren erhältlich ist.
Die Erfindung wird nun anhand von Figuren, Testmethoden und Beispielen näher erläutert.

FIGUREN:

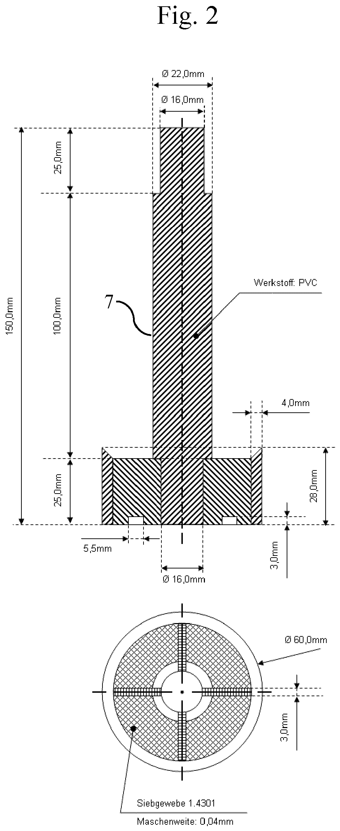

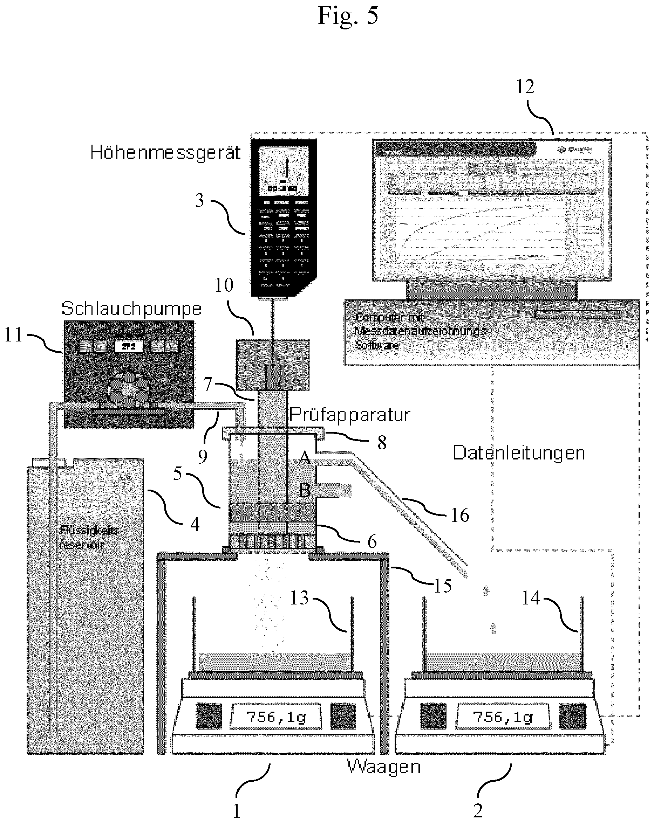

Es zeigt die Figur 1 den zur Bestimmung der APC-Werte eingesetzten Messtopf 6 mit den jeweiligen Maßen (in mm).
Es zeigt die Figur 2 den zur Bestimmung der APC-Werte eingesetzten Stempel 7 mit den jeweiligen Maßen (in mm), welcher in den in der Figur 1 gezeigten Messtopf 6 eingesetzt wird.
Es zeigt die Figur 3 den aus PVC gefertigten Deckel 8, der auf den Messtopf 6 aufgesetzt wird.
Es zeigt die Figur 4 das Gewicht10, welches auf den Stempel 7 aufgesetzt wird. Die Höhe bzw. Breite des Gewichtes 10 richten sich nach dem Gewicht des verwendeten Materials. Die Gesamtbelastung durch Stempel 7 und Gewicht 10 beträgt 594 g $\pm$ 5 g (entspricht einer Belastung von 21 g/cm$^2$ $\pm$ 0,2 g/cm$^2$. Die Breite des Gewichtes soll 70 mm nicht überschreiten.
Es zeigt die Figur 5 die Versuchsanordnung bei der Bestimmung der APC-Werte.

TESTMETHODEN

Bestimmung der APC-Werte

Die Bestimmung der APC-Werte erfolgte mittels der in Figur 5 gezeigten Messanordnung.

### 1. Prinzip der Messung

Bei der Bestimmung der APC-Werte werden 1,800 g ($\pm$ 0,005 g) des zu analysierenden, teilchenförmigen, absorbierenden Polymermaterials (nachfolgend als "SAP" bezeichnet) in den in der Figur 1 gezeigten Messtopf 6 mit Siebgewebe im Boden eingestreut und gleichmäßig auf dem Siebboden verteilt. Anschließend wird der in der Figur 2 gezeigte Stempel in den Messtopf eingeführt und, wie in der Figur 5 gezeigt, mit einem solchen Gewicht 10 (siehe Figur 4) belastet, dass auf das SAP-Material ein Druck von 21,0 g/cm$^2$ $\pm$ 0,2 g/cm$^2$ (Gesamtgewicht aus Stempel und Gewicht = 594 g $\pm$ 5 g). Die Zylindereinheit (Messtopf 6, SAP, Stempel 7 und Gewicht 10) wird auf ein Stativtisch 15 mit kreisrundem Ausschnitt gestellt, wie dies in der Figur 5 gezeigt ist. Der Stativtisch 15 steht über einer Waage 1. Über eine Schlauchpumpe 11 wird die Testlösung mit konstantem Durchfluss in die Zylindereinheit gegeben. Durchlaufende Flüssigkeit wird in dem Auffanggefäß 13 unter dem Stativtisch 15 (auf einer Waage 1) fortlaufend gemessen. In dem Messtopf 6 befinden sich in vorher definierter Höhe Abläufe A und B (der untere Ablauf B in dem in Figur 1 gezeigten Messtopf 6 ist, wie in der Figur 5 kenntlich gemacht, verschlossen). Bei Ausbildung der Gelschicht kann sich eine Flüssigkeitssäule bis zur Höhe des Ablaufes A einstellen. Danach läuft überschüssige Flüssigkeit über den Ablauf A ab. Diese Flüssigkeit wird über ein etwa 10 cm langes Schlauchstück 16 ebenfalls in ein Auffanggefäß 14 geleitet und auf einer Waage 2 fortlaufend gemessen. Während der gesamten Messung wird die Höhe der sich bildenden Gelschicht ebenfalls fortlaufend gemessen. Alle Messungen (Waagen, Höhenmessung) werden automatisch erfasst und gespeichert.

Damit werden, bei Konstant gewählten Messbedingungen (Menge an eingewogenem SAP in g, auf den SAP wirkender Druck in g/cm$^2$, Zustrom an Testlösung in die Zylindereinheit in g/sec) kontinuierlich die Folgenden Messdaten ($\sigma$1) bis ($\sigma$3) erfasst:

($\sigma$1) Änderung der Höhe der sich bildenden Gelschicht durch das Höhenmessgerät 3;
($\sigma$2) Änderung des Gewichtes auf der Waage 1 links in der Figur 5 durch die durch die Gelschicht laufende Testflüssigkeit;
($\sigma$3) Änderung des Gewichtes auf der Waage 2 rechts in der Figur 5 durch die durch über den Ablauf A ablaufende Testflüssigkeit.

### 2. Verwendete Messgeräte

- 2 Waagen (1, 2) mit einer Genauigkeit von min 0,1g, ausgestattet mit einer PC-Schnittstelle (z. B. Sartorius CP 8201).

- Höhenmessgerät 3 mit einer Genauigkeit von min. 0,1 mm und einer Messlänge von min. 30mm, ausgestattet mit einer PC-Schnittstelle (z. B Tesa Digico 2).

- Flüssigkeitsreservoir 4 (mindestens entsprechend der Flüssigkeitsmenge, welche durch die Testparameter, z. B. 30min mit 2g/s = 3.600g, vorgegeben ist).

- Prüfapparatur-Apparatur 5 mit Belastung (siehe auch Figuren 1 und 2).

- Transparenter Messtopf 6 aus Plexiglas gemäß Figur 1, (d1 ($\varnothing$innen) = 60,3 mm $\pm$ 0 mm, h = 100 mm $\pm$ 0,5 mm) mit 2 Ausläufen (von denen nur der obere genutzt und der untere während der Messung verschlossen wird) in h1* = 55 mm und h2* = 83 mm ($\varnothing$innen = 10 mm) (*mittlere Höhe der Ausläufe). Die untere Seite des Messtopfes ist mit einem rostfreien Stahlsiebgewebe (400 mesh = 36 $\mu$m) versehen.

- Kunststoffstempel 7 (d2 ($\varnothing$außen) = 60 mm $\pm$0 mm) aus PVC gemäß Figur 2 (d1 - d2 = 0,3 mm und h = 150 mm). Die untere Seite des Kunststoffstempels 7 ist mit einem rostfreien Stahlsiebgewebe (400 mesh = 36 $\mu$m) versehen. Die Höhe des unteren Teils des Kunststoffstempels 7 beträgt 28 mm. Es befinden sich 4 Stege in diesem Teil, welche zur Stabilisation des Siebgewebes beitragen. Jeder Steg hat im unteren Bereich (also in dem Bereich, der sich im Kontakt mit dem Stahlsiebgewebe befindet) einen ca. 3 x 5,5 mm Durchlass, damit gewährleistet ist, das die Flüssigkeit sich gleichmäßig verteilen kann.

- Ein passgenauer Deckel 8 mit Ausschnitten für den Kunststoffstempel 7 und die Flüssigkeitszuführung 9 (siehe Figur 3).

- Gewicht 10, welches auf den Kunststoffstempel 7 aufgesetzt wird (siehe Figur 4). Kunststoffstempel 7 und Gewicht 10 müssen zusammen das definierte Gewicht erreichen. Standard ist hierbei eine Gesamtmasse von 594 g $\pm$ 5 g (entspricht einer Belastung von etwa 21 g/cm$^2$ $\pm$ 0,2 g/cm$^2$, was einem Druck von etwa 0,3 psi entspricht).

- Flüssigkeitspumpe 11 (z.B. Schlauchpumpe) mit einer Förderleistung von 30 mL/min bis 400 mL/min, z.B. Ismatec MCP Standard mit Pumpenkopf Ismatec SB-2V und Schlauch "Tygon Standard" (ID 4,8, WS 1,6, AD 8,0).

- Computersystem 12 mit entsprechender Software zum Aufzeichnen der Waagenanzeige & Höhenmessungen.

- Auffanggefäße 13, 14 für Flüssigkeit (min. 3,6 L).

- Stativtisch 15 mit einer kreisrunden Aussparung ($\varnothing$ 6 cm, entspricht genau der Breite des Siebgewebes im Messtopf). Um diese Aussparung ist ein ca. 2 cm hoher Kunststoffring ($\varnothing$ 7,2 cm, entspricht in etwa dem äußeren Durchmesser des Messtopfes) zentrisch angebracht (dadurch wird die Zylindereinheit während der Messung fixiert, damit die Flüssigkeit ungehindert durchlaufen kann und die Höhenmessung immer an der gleichen Stelle erfolgt).

3. Durchführung der Messung

Es ist mindestens eine 2-fach-Bestimmung durchzuführen.

Vorbereitende Arbeiten:

- Das Flüssigkeitsreservoir 4 wird befüllt, so das ausreichend Testflüssigkeit (0,9%ige NaCl-Lösung) zur Verfügung steht. Die Temperatur der Testflüssigkeit beträgt 23°C $\pm$ 2,0 °C (Raumtemperatur = 23°C $\pm$ 2,0 °C, Luftfeuchtigkeit 55 % $\pm$ 15 %).

- Die Schlauchpumpe 11 wird entsprechend der beabsichtigten Zuflussrate justiert. Dazu kann entweder, soweit geeignet, das System zur Messwertaufzeichnung genutzt werden oder alternativ kann auch mittels Stoppuhr und Becherglas o.ä. die erforderliche Menge bestimmt werden. Standartzuflussraten sind 1 bzw. 2 g/s.

- Das System zur Messwertaufzeichnung wird aufgebaut. Es können alle Systeme verwendet werden, welche Messdaten von Waagen und Höhenmessgeräten zeitgesteuert aufzeichnen können. Beispielsweise kann ein solches System aus einem Computer mit Windowsbetriebssystem (ab XP), Microsoft Excel (ab Version 6), einer 4-fach Schnittstellenkarte RS232 und einem programmierten Visual Basic for Application Modul bestehen. Das System muss in der Lage sein, alle 3 Messwertabfragen ($\sigma$1) bis ($\sigma$3) in einem Zeitfenster von maximal 0,5 Sekunden durchzuführen. Die Waagen 1 und 2 und das Höhenmessgerät 3 werden mit dem Computer 12 verbunden (In dem Programm zur Messwertaufzeichnung müssen die entsprechenden Einstellungen (Baudrate, Parität etc.) zu den Waagen 1 und 2 bzw. zu dem Höhenmessgerät 3 vorgenommen werden.

Messung:

- Die zu analysierende SAP-Probe ist gut zu durchmischen. Das zu entnehmende Testmaterial sollte frei von Klumpen und Verunreinigungen sein. Es werden 1,800 g $\pm$ 0,005 g SAP in die Prüfapparatur eingewogen. Der SAP wird gleichmäßig auf dem Siebboden verteilt.

- Die SAP-Probe wird mittels des Stempels 7 und dem Gewicht 10 mit 21,0 g/cm$^2$ $\pm$ 0,2 g/cm$^2$ belastet und in den Ausschnitt des Stativtisches 15 gestellt. Das mit einem Auslauf versehene Schlauchende der Schlauchpumpe (Flüssigkeitszuführung 9) wird mit Länge von 2-3 cm durch die Zugabeöffnung der Abdeckplatte 8 der Prüfapparatur gesteckt.

- Das Höhenmessgerät 3 wird mit der Messstange zentriert auf den Stempel 7 der Prüfapparatur installiert. Während der Testdauer von 30 Minuten werden alle 10 Sekunden die Messwerte abgefragt und mit der jeweiligen Zeit aufgezeichnet.

- Die Waagen 1 und 2 werden tariert und das Höhenmessgerät 3 genullt.

- Das System zur Messwertaufzeichnung wird initialisiert.

- Unmittelbar, spätestens jedoch 0,5 Sekunden nach der Initialisierung des Systems zur Messwertaufzeichnung wird die Zugabe mittels der Schlauchpumpe 11 gestartet.

- Nach Ablauf der Testdauer wird die Schlauchpumpe 11 abgestellt.

4. Auswertung

Aus den Datenreihen Höhe (ht) in mm zur Zeit (t) in s ($\sigma 1$), Durchfluss (Fl.th.$_t$) in g zur Zeit (t) in s ($\sigma 2$) und Überlauf (Fl.ov.t) in g zur Zeit (t) in s ($\sigma 3$) lassen sich folgende weitere Werte berechnen (jeweils zur Zeit t):

Vzu$_t$ in g:            Flüssigkeitsvolumenzugabe zur Zeit t;

V$_t$ in cm$^3$:          das Gelvolumen zur Zeit t (Grundfläche des Messtopfes 6 = 28,27cm$^2$):

Formel:

$$V_t[cm^3] = h_t[mm]/10 * 28,27\,cm^2$$

$\Delta$V$_t$ in cm$^3$:         der Gelvolumenzuwachs innerhalb 10s zur Zeit t (Grundfläche des Messtopfes 6 = 28,27cm$^2$:

Formel:

$$\Delta V_t[cm^3] = (h_t[mm] - h_{t-10}[mm])/10 * 28,27\,cm^2$$

QI$_t$:              Quellindex (Einheitenlos) zur Zeit t (E = Einwaage SAP in g; für eine 0,9 %ige NaCl-Lösung wird 1 cm$^3$ = 1 g gesetzt):

Formel:

$$QI_t = \Delta V_t[cm^3]/(E[g] * 10)$$

$\Delta$Fl.th.$_t$ in g/s:   Durchflussrate gemittelt über 10s zur Zeit t:

Formel:

$$\Delta Fl.th._t[g/s] = (Fl.th._t[g] - Fl.th._{t-10}[g])/10s$$

Vü$_t$ in g:          Volumenüberstand im APC-Messtopf:

Formel:

$$Vü_t[cm^3 = g] = Vzu_t[g] - V_t[cm^3] - Fl.ov._t[g] - Fl.th._t[g]$$

h(Vü)$_t$ in mm:      theoretische (berechnete) Höhe der Flüssigkeit im APC-Topf (mit Berücksichtigung der Fläche des Stempel = 5 cm$^2$; effektive Fläche = 28,27cm$^2$-5cm$^2$=23,27cm$^2$):

Formel:

$$h(V\ddot{u})_t[mm] = (h_t[mm]/10 + (V\ddot{u}_t[g]/23,27cm^2))*10$$

$PI_t$: Permeabilitätsindex (Einheitenlos) zur Zeit t = 20s... 120s:

Formel:

$$PI_t = \frac{\Delta Fl.th._t[cm^3/\sec] \times h_t[cm]}{981(cm/\sec^2) \times h(V\ddot{u})_t[cm] \times 28,27cm^2 * 10^{-7}\sec}$$

$APC_{i \times 10\,sec}$: $APC_{i \times 10\,sec}$-Wert zur Zeit T = 20s... 120s (i = 2 bis 12). Aus den vorstehend beschriebenen Werten wird weitergehend noch der $APC_{max}$-Wert (also der höchste der 11 bestimmten APC-Werte), der APC-Durchschnittswert $APC_{mittel}$ und die Summe aller APC-Werte ($APC_{sum}$) bestimmt.

Formeln:

$$APC_{i \times 10\,\sec} = QI_{i \times 10\,\sec}^{\,2} * PI_{i \times 10\,\sec}$$

$$APC_{mittel} = \frac{1}{11}\sum_{i=2}^{12} APC_{i \times 10\,\sec}$$

$$APC_{sum} = \sum_{i=2}^{12} APC_{i \times 10\,\sec}$$

BEISPIELE

Vergleichsbeispiel (nicht erfindungsgemäß)

In 1972,90 g einer wässrigen Lösung aus Natriumacrylat mit einem Neutralisationsgrad von 70 mol-% (bez. Acrylsäure) sowie einer gesamt Monomerkonzentration von 39,37 % werden 1,770 g Polyethylenglycol-300-diacrylat (0,2 % bez. auf Acrylsäure/Estergehalt = 72 %) und 2,560 g Polyethylenglycol-(440)monoallyl-etheracrylat (0,4% bez. auf Acrylsäure/Estergehalt=100 %) als Vernetzer gelöst. Die Monomerlösung wird in einem Kunststoff-Polymerisationsgefäß für 30 Minuten mit Stickstoff durchspült, um den gelösten Sauerstoff zu entfernen. Bei einer Temperatur von 4 °C wird die Polymerisation durch die aufeinanderfolgende Zugabe von 0,6 g Natriumperoxodisulfat in 10 g dest. Wasser, 0,14 g 35 %ige Wasserstoffperoxidlösung in 10 g dest. Wasser und 0,03 g Ascorbinsäure in 2 g dest. Wasser gestartet. Nachdem die Endtemperatur (ca. 100 °C) erreicht ist, wird das Gel mit einem Fleischwolf zerkleinert und 2 h bei 150 °C in einem Umlufttrockenschrank getrocknet. Das getrocknete Produkt wird grob zerstoßen, gemahlen und auf eine Korn-fraktion von 150-710 μm abgesiebt. Der erhaltene Bulk wird durch Siebung in einzelne Kornfraktionen aufgetrennt und wie folgt zu einer synthetisch hergestellten PSD zusammengemischt:

$$>150\,\mu m = 15\,\% / >300\,\mu m = 48\,\% / >500\,\mu m = 32\,\% / >600\,\mu m = 5\,\%.$$

Die Nachvernetzung der so erhaltenen Vorprodukte erfolgt durch die Beschichtung mit einer Lösung bestehend aus Ethylencarbonat/Wasser/Aluminiumlactat/Aluminiumsulfat im Verhältnis 1/3/0,4/0,3% bez. Auf 100g Vorprodukt sowie anschließender Nacherhitzung im Trockenschrank bei 170°C, 90 Minuten.

Beispiel 1 (erfindungsgemäß)

In 963,60 g einer wässrigen Lösung aus Natriumacrylat mit einem Neutralisationsgrad von 70 mol % (bez. Acrylsäure) sowie einer gesamt Monomerkonzentration von 40,30 % werden 0,775 g Polyethylenglycol-300-diacrylat (0,20 % bez. auf Acrylsäure/Estergehalt = 83%) und 1,639 g Polyethylenglycol-(440)mono-allyletheracrylat (0,40 % bez. auf Acrylsäure/Estergehalt = 78 %) als Vernetzer gelöst. Dieser Lösung werden anschließend 9,6 g einer 10 %igen wässrigen Lösung des Comonomers Pluriol A 23 R (Fa. BASF) zugegeben und die Monomerlösung wird in einem Kunststoff-Polymerisationsgefäß für 30 Minuten mit Stickstoff durchspült, um den gelösten Sauerstoff zu entfernen. Bei einer Temperatur von 4 °C werden 2 g Soda leicht calciniert (Fa. Solvay) als Treibmittel zugesetzt und die Polymerisation durch die aufeinanderfolgende Zugabe von 0,3 g Natriumperoxodisulfat in 10 g dest. Wasser, 0,07 g 35 %ige Wasserstoffperoxidlösung in 10 g dest. Wasser und 0,015 g Ascorbinsäure in 2 g dest. Wasser gestartet. Nachdem die Endtemperatur (ca. 100 °C) erreicht ist, wird das Gel mit einem Fleischwolf zerkleinert und 2 h bei 150 °C in einem Umlufttrockenschrank getrocknet. Das getrocknete Produkt wird grob zerstoßen, gemahlen und auf eine Kornfraktion von 150-710 μm abgesiebt. Der erhaltene Bulk wird durch Siebung in einzelne Kornfraktionen aufgetrennt und wie folgt zu einer synthetisch hergestellten PSD zusammengemischt:

$$>150 \ \mu m = 15 \ \% \ / >300 \ \mu m = 48 \ \% \ / >500 \ \mu m = 32 \ \% \ / >600 \ \mu m = 5 \ \%.$$

Die Nachvernetzung der so erhaltenen Vorprodukte erfolgt durch die Beschichtung mit einer Lösung bestehend aus Ethylencarbonat/Wasser/Aluminiumlactat/Aluminiumsulfat im Verhältnis 1/3/0,4/0,3 % bez. Auf 100 g Vorprodukt sowie anschließender Nacherhitzung im Trockenschrank bei 170°C, 90 Minuten.

Beispiel 2 (erfindungsgemäß)

In 1962,4 g einer wässrigen Lösung aus Natriumacrylat mit einem Neutralisationsgrad von 70 mol-% (bezogen auf Acrylsäure) sowie einer gesamt Monomerkonzentration von 39,6 Gew.-% werden 1,529 g Polyethylenglykol-300-diacrylat (Estergehalt = 83,7 %; 0,2 Gew.-% bezogen auf Acrylsäure) und 3,303 g Polyethylenglycol-440-monoallyletheracrylat (0,40 Gew. % bezogen auf Acrylsäure/Estergehalt = 77,5%) gelöst. Als Treibmittel werden im späteren Verlauf 10,0 g Natriumcarbonat, hergestellt im Fließbett-Spray-Granulationsverfahren, mit einer Partikelgröße von 400-600 μm (0,5 Gew. % bezogen auf Ansatzgröße) hinzugegeben. Die Monomerlösung wird für 15-30 Minuten mit Stickstoff durchspült, um den gelösten Sauerstoff zu entfernen. Bei einer Temperatur von 20 °C wird die Monomerlösung, die mit 0,6 g Natriumperoxodisulfat in 10 g destilliertem Wasser und 0,14 g 35 %ige Wasserstoffperoxidlösung in 10 g destilliertem Wasser versetzt wird, unter Inertgasgegenstrom und laufenden Wellen in den List Batch CRP 2.5 Kneter überführt. Die eingestellten Sollwerte und damit die angestrebten Parameter während der Reaktion im zweiwellig, gleichläufig drehenden, diskontinuierlich betriebenen Knetreaktor, bzw. die der Peripheriegeräte sind: Temperatur des Öles im Heizkreislauf des Reaktormantels: 75°C; Drehzahl der Wellen: 60 Umdrehungen pro Minute. Nach Transfer der Monomerlösung in den Reaktorinnenraum wird das Natriumcarbonat hinzugegeben. Nach 30 Sekunden Einrührphase wird 0,030 g Ascorbinsäure in 2 g destilliertem Wasser in den Reaktionsinnenraum gegeben und die exotherme Reaktion gestartet. Der Sollwert des Öles im Heizkreislauf des Reaktormantels wird auf 100°C gestellt. Nach Ablauf von 5 Minuten wird der Heizkreislauf ausgeschaltet. Nach insgesamt 10 Minuten werden die gleichläufig drehenden Wellen gestoppt, der Reaktor geöffnet, das entstandene Hydrogel entnommen und bei 150°C für 2 Stunden im Umlufttrockenschrank getrocknet. Das getrocknete Produkt wird zerstoßen, mit einer Schneidmühle der Firma Retsch und Schneidkranz mit Lochgröße 2mm gemahlen, durch Siebung in einzelne Kornfraktionen aufgetrennt und wie folgt zu einer synthetischen Kornverteilung zusammengemischt:

$$>150 \ \mu m = 15 \ \% \ / >300 \ \mu m = 48 \ \% \ / >500 \ \mu m = 32 \ \% \ / >600 \ \mu m = 5 \ \%.$$

Die Nachvernetzung erfolgt durch eine Beschichtung mit einer Lösung bestehend aus Ethylencarbonat/Wasser/Aluminiumlactat/Aluminiumsulfat im Verhältnis 1/4/0,4/0,3% bezogen auf 100g Vorprodukt sowie anschließender Nacherhitzung im Trockenschrank bei 175°C, 90 Minuten.

Beispiel 4

Von dem im Vergleichsbeispiel und den Beispielen 1 und 2 erhaltenen Polymerisaten werden der $APC_{max}$-Wert sowie der $APC_{sum}$-Wert mittels des hierin beschriebenen Testverfahrens ermittelt. Die Ergebnisse sind der Tabelle 3 zu entnehmen. In den Tabellen 1 und 2 sind exemplarisch die bei der Bestimmung der APC-Parameter kontinuierlich während der Messdauer von 120 Sekunden ermittelten Messwerte bzw. der hieraus rein rechnerisch abgeleiteten Messdaten aus der APC-Analyse des Polymers aus dem Vergleichsbeispiel zusammengestellt.

Tabelle 1: die unmittelbar ermittelten Messwerte ($\sigma$1), ($\sigma$2) und ($\sigma$3)

| Zeit t | ht | Zeit t | Fl.th.t | Zeit t | Fl.ov.t |
|--------|------|--------|---------|--------|---------|
| [s] | [mm] | [s] | [g] | [s] | [g] |
| 0 | 0,00 | 0 | 0,50 | 0 | 0,01 |
| 10 | 0,28 | 10 | 4,65 | 10 | 0,01 |
| 20 | 1,22 | 20 | 15,35 | 20 | 0,00 |
| 30 | 2,15 | 30 | 27,77 | 30 | 0,00 |
| 40 | 2,96 | 40 | 41,20 | 40 | 0,00 |
| 50 | 3,65 | 50 | 55,07 | 50 | 0,00 |
| 60 | 4,27 | 60 | 69,80 | 60 | 0,00 |
| 70 | 4,87 | 70 | 85,12 | 70 | 0,00 |
| 80 | 5,41 | 80 | 100,63 | 80 | 0,00 |
| 90 | 5,92 | 90 | 116,13 | 90 | 0,01 |
| 100 | 6,42 | 100 | 131,55 | 100 | 0,01 |
| 110 | 6,88 | 110 | 147,15 | 110 | 0,02 |
| 120 | 7,31 | 120 | 162,88 | 120 | 0,01 |

Tabelle 2: aus den Messwerten der Tabelle 1 rechnerisch ermittelte Messgrößen

| Zeit | $DV_t$ | $Ql_t$ | DFl. th. | $Vzu_t$ | $V_t$ | $Vü_t$ | $h(Vü)_t$ | $Pl_t$ | APC-Wert[1] |
|------|--------|--------|----------|---------|-------|--------|-----------|--------|-------------|
| (sec) | [cm$^3$] | | [g/s] | [g] | [cm$^3$] | [cm$^3$] | [mm] | | |
| 0 | 0,00 | 0,00 | 0,50 | 0 | 0,01 | 0,00 | 0,00 | | |
| 10 | 0,78 | 0,04 | 0,42 | 20 | 0,79 | 14,55 | 6,53 | 6,42 | |
| 20 | 2,67 | 0,15 | 1,07 | 40 | 3,46 | 21,19 | 10,33 | 45,72 | 1,01 |
| 30 | 2,62 | 0,15 | 1,24 | 60 | 6,08 | 26,14 | 13,39 | 72,03 | 1,53 |
| 40 | 2,28 | 0,13 | 1,34 | 80 | 8,36 | 30,44 | 16,04 | 89,31 | 1,43 |
| 50 | 1,97 | 0,11 | 1,39 | 100 | 10,33 | 34,59 | 18,52 | 98,69 | 1,19 |
| 60 | 1,75 | 0,10 | 1,47 | 120 | 12,08 | 38,11 | 20,65 | 109,86 | 1,04 |
| 70 | 1,67 | 0,09 | 1,53 | 140 | 13,75 | 41,12 | 22,54 | 119,25 | 1,03 |
| 80 | 1,53 | 0,08 | 1,55 | 160 | 15,28 | 44,09 | 24,35 | 124,10 | 0,90 |
| 90 | 1,46 | 0,08 | 1,55 | 180 | 16,75 | 47,12 | 26,17 | 126,36 | 0,84 |
| 100 | 1,39 | 0,08 | 1,55 | 200 | 18,14 | 50,30 | 28,03 | 127,54 | 0,76 |
| 110 | 1,30 | 0,07 | 1,56 | 220 | 19,44 | 53,39 | 29,82 | 129,55 | 0,68 |
| 120 | 1,23 | 0,07 | 1,57 | 240 | 20,67 | 56,45 | 31,57 | 131,22 | 0,61 |
| [1] Als Mittelwert einer vierfach-Bestimmung | | | | | | | | | |

Tabelle 3: APC-Parameter der Polymerisate aus dem Vergleichsbeispiel und den Beispielen 1 und 2

| Polymer | $APC_{max}$ | $APC_{sum}$ | CRC [g/g] | AAP [g/g] |
|---|---|---|---|---|
| Vergleichsbeispiel | 1,53 | 11,0 | | |
| Beispiel 1 | 7,01 | 37,3 | 26,1 | 25,3 |
| Beispiel 2 | 3,47 | 18,9 | 25,9 | 25,2 |

Beispiel 5

Aus dem im Vergleichsbeispiel und den Beispielen 1 und 2 erhaltenen Polymerisaten wird ein absorbierender Kern hergestellt.

Ein zugeschnittenes Nonwoven (Art.-Nr. 00028710 der Firma Freudenberg) mit den Maßen von 12 x 12cm, wird mittels einer pneumatischen Schmelzklebstoffpistole der Firma Bühnen, Typ HB 710 Spray inkl. Dralldüsensatz Standard ⌀ 1,5 mm, betrieben bei 4 bar Druckluft, für 30 Sekunden mit Kleber (Dispomelt CS 22 der Firma Henkel) gleichmäßig besprüht. Die Sprühpistole wird zuvor auf eine Schmelztemperatur von 160°C eingestellt. Es werden genau 5 g SAP gleichmäßig auf eine zuvor markierte / zentrierter Fläche von 10 × 10 cm aufgestreut. Ein weiteres Nonwoven mit den gleichen Maßen wird auf die gleiche Weise mit dem Kleber besprüht und dann mit der Kleberseite nach unten auf die SAP-Lage gelegt, so dass ein absorbierenden Kern mit folgender Schichtenlage erhalten wurde:

Nonwoven

Kleber

SAP

Kleber

Nonwoven.

Danach erfolgte ein Anpressen der Schichten mittels einer Handwalze. Anschließend wurde der absorbierende Kern zum Trocknen des Klebers für eine Stunde gelagert, bevor er vermessen wurde.

Zur Bestimmung der Absorptionseigenschaften des absorbierenden Kerns wurde der zu prüfende Kern gewogen und auf ein Drahtgeflecht mit einer Maschenweite von 9 × 9 mm (Drahtstärke 1,5 mm) und einem Aussenmaß von 12 × 12 cm gelegt, welches mittels vier etwa 1,2 cm hoher Metallfüße, die an den Ecken des Drahtgeflechts positioniert waren, in einer Petrischale platziert wurde. Der Absorbierende Kern wird mit einer Testplatte (10 × 10 cm) mit einem Gewicht von 200 g belastet. Mittig ist diese Testplatte mit einem Zugaberohr versehen, welches eine Höhe von 20 cm und einen inneren Durchmesser von 20 mm aufweist. Durch dieses Zugaberohr kann Testflüssigkeit durch die Testplatte hindurch auf den absorbierenden Kern geleitet werden, während sich dieser unter dem Druck der Testplatte befindet.

Es werden viermal nacheinander jeweils 25 g einer 0,9%igen NaCl-Lösung, welche mit Säurefuchsin leicht eingefärbt wird, in das Zugaberohr eingefüllt (Wartezeit zwischen den einzelnen Zugaben: 15 Minuten). Vor der ersten Zugabe wird der absorbierende Kern gewogen (w1). Die Testlösung wird jeweils über das Zugaberohr zugeführt. Nach vollständiger Flüssigkeitsaufnahme (4 × 25 g = 100 g) erfolgt eine erneute Wiegung des absorbierenden Kerns (w2). Die Leakage-Menge (1) kann rechnerisch aus der Differenz zwischen der zugegebenen Flüssigkeitsmenge w0 und der Differenz aus Gewicht des absorbierenden Kerns nach und vor der Absorption ermitteln werden($1 = w_0-(w2-w1)$).

Dabei wurden folgende Messwerte ermittelt:

Tabelle 4: Leakage-Werte der Polymerisate aus dem Vergleichsbeispiel und den Beispielen 1 und 2 in einem absorbierenden Kern

| Polymer | Leakage [g] nach viermaliger Zugabe |
|---|---|
| Vergleichsbeispiel | 18,7 |
| Beispiel 1 | 8,4 |
| Beispiel 2 | 12,9 |

## Patentansprüche

**1.** Verfahren zur Herstellung eines absorbierenden Kerns, welcher eine obere Substratschicht, eine untere Substratschicht und eine zwischen der oberen und der unteren Substratschicht angeordnete Absorptionsschicht beinhaltet, wobei die Absorptionsschicht ein teilchenförmiges absorbierendes Polymermaterial und weniger als 0,1 g Cellulosefasern pro Gramm des teilchenförmigen absorbierenden Polymermaterials umfasst,
beinhaltend die Verfahrensschritte:

A) Bereitstellen eines teilchenförmigen absorbierenden Polymermaterials;
B) Einarbeiten des teilchenförmigen absorbierenden Materials in einen absorbierenden Kern;

wobei das bereitgestellte teilchenförmige, absorbierendes Polymermaterial die folgenden Eigenschaften aufweist:

i) einen maximalen $APC_{i \times 10\,sec}$-Wert $\geq 1,6$ für mindestens eine Zahl i ausgewählt aus der Gruppe der ganzen Zahlen von 2 bis 12 (=APCmax-Wert);
ii) einen Wert für die Summe aller $APC_{i \times 10\,sec}$-Werte $\geq 12$ für alle Zahlen i aus der Gruppe der ganzen Zahlen von 2 bis 12 (=$APC_{sum}$-Wert);

wobei der $APC_{i \times 10\,sec}$-Wert wie folgt definiert ist:

$$APC_{i \times 10\,sec}\text{-Wert} = QI_{i \times 10\,sec}\text{-Wert}^2 \times PI_{i \times 10\,sec}\text{-Wert}$$

wobei

- der $QI_{i \times 10\,sec}$-Wert der Wert des gemäß der hierin beschriebenen Testmethode $i \times 10$ Sekunden nach Zugabe der 0,9 gew.-%igen NaCl-Lösung bestimmten Quellindex und
- der $PI_{i \times 10\,sec}$-Wert der Wert des gemäß der hierin beschriebenen Testmethode $i \times 10$ Sekunden nach Zugabe der 0,9 gew.-%igen NaCl-Lösung bestimmten Permeabilitätsindex

sind;
**dadurch gekennzeichnet,**
**dass** das Bereitstellen des teilchenförmigen absorbierenden Polymermaterials erfolgt durch:

Selektion eines geeigneten teilchenförmigen absorbierenden Polymermaterials auf der Basis der Ergebnisse der Bestimmung des Produktes aus (Quellindex)$^2$ und (Permeabilitätsindex) (= APC-Wert) für mindestens einen Zeitpunkt $t_i$ nach Zugabe einer Flüssigkeit zu dem teilchenförmigen absorbierenden Polymergebilde, wobei das Produkt aus Quellindex$^2$ und Permeabilitätsindex (= APC-Wert) für mindestens 11 verschiedene Zeitpunkte $t_i$ bestimmt und die Selektion des teilchenförmigen absorbierenden Polymermaterials erfolgt auf der Basis der Summe aller erhaltenen APC-Werte.

## Claims

**1.** Process for producing an absorbent core containing an upper substrate layer, a lower substrate layer and an absorption layer arranged between the upper and the lower substrate layers, wherein the absorption layer comprises a particulate absorbent polymer material and less than 0.1 g of cellulose fibres per gram of particulate absorbent polymer material,
containing the process steps of:

A) providing a particulate absorbent polymer material;
B) incorporating the particulate absorbent material in an absorbent core;

wherein the particulate absorbent polymer material provided has the following properties:

i) a maximum $APC_{i \times 10\,sec}$ value $\geq 1.6$ for at least one number i selected from the group of integers from 2 to 12 (= $APC_{max}$ value) ;
ii) a value $\geq 12$ for the sum total of all $APC_{i \times 10\,sec}$ values for all numbers i from the group of integers from 2 to

12 (= APC<sub>sum</sub> value) ;

wherein the APC$_{i \times 10\,sec}$ value is defined as follows:

$$\text{APC}_{i \times 10\ sec}\ value = \text{QI}_{i \times 10\ sec}\ value^2 \times \text{PI}_{i \times 10\ sec}\ value$$

where

- the QI$_{i \times 10\,sec}$ value is the value of the swell index determined as per the herein described test method i $\times$ 10 seconds after adding the 0.9% by weight NaCl solution, and
- the PI$_{i \times 10\,sec}$ value is the value of the permeability index determined as per the herein described test method i $\times$ 10 seconds after adding the 0.9% by weight NaCl solution;

**characterized in that**
the particulate absorbent polymer material is provided by:

selecting a suitable particulate absorbent polymer material on the basis of the results of determining the product of (swell index)$^2$ and (permeability index) (= APC value) for at least one time t$_i$ after adding a fluid to the particulate absorbent polymer structure;
where the product of swell index$^2$ and permeability index (= APC value) is determined for at least 11 different times t$_i$ and the particulate absorbent polymer material is selected on the basis of the sum total of all APC values obtained.

**Revendications**

1. Procédé de fabrication d'un noyau absorbant, qui contient une couche de substrat supérieure, une couche de substrat inférieure et une couche d'absorption agencée entre la couche de substrat supérieure et inférieure, la couche d'absorption comprenant un matériau polymère absorbant particulaire et moins de 0,1 g de fibres de cellulose par gramme du matériau polymère absorbant particulaire,
contenant les étapes de procédé suivantes :

A) la préparation d'un matériau polymère absorbant particulaire ;
B) l'incorporation du matériau absorbant particulaire dans un noyau absorbant ;

le matériau polymère absorbant particulaire préparé présentant les propriétés suivantes :

i) une valeur APC$_{i\,x\,10\,s}$ maximale $\geq$ 1,6 pour au moins un nombre i choisi dans le groupe constitué par les nombres entiers allant de 2 à 12 (= valeur APCmax) ;
ii) une valeur pour la somme de toutes les valeurs APC$_{i\,x\,10\,s}$ $\geq$ 12 pour tous les nombres i du groupe constitué par les nombres entiers allant de 2 à 12 (= valeur APC$_{somme}$) ;

la valeur APC$_{i\,x\,10\,s}$ étant définie de la manière suivante :

$$\text{valeur APC}_{i\,x\,10\,s} = \text{valeur QI}_{i\,x\,10\,s}^2 \times \text{valeur PI}_{i\,x\,10\,s}$$

- la valeur QI$_{i\,x\,10\,s}$ étant la valeur de l'indice de gonflement déterminé selon la méthode d'essai décrite dans le présent document i x 10 secondes après l'ajout de la solution de NaCl à 0,9 % en poids et
- la valeur PI$_{i\,x\,10\,s}$ étant la valeur de l'indice de perméabilité déterminé selon la méthode d'essai décrite dans le présent document i x 10 secondes après l'ajout de la solution de NaCl à 0,9 % en poids ;

**caractérisé en ce que**
la préparation du matériau polymère absorbant particulaire a lieu par :

sélection d'un matériau polymère absorbant particulaire approprié sur la base des résultats de la détermination du produit de (l'indice de gonflement)$^2$ et de (l'indice de perméabilité) (= valeur APC) pour au moins un temps $t_i$ après l'ajout d'un liquide à la structure polymère absorbante particulaire,

le produit de l'indice de gonflement$^2$ et de l'indice de perméabilité (= valeur APC) étant déterminé pour au moins 11 temps $t_i$ différents et la sélection du matériau polymère absorbant particulaire ayant lieu sur la base de la somme de toutes les valeurs APC obtenues.

# Fig. 1

# Fig. 2

Ø 22,0mm
Ø 16,0mm
25,0mm
150,0mm
100,0mm
7
Werkstoff: PVC
4,0mm
25,0mm
28,0mm
5,5mm
3,0mm
Ø 16,0mm
Ø 60,0mm
3,0mm
Siebgewebe 1.4301
Maschenweite: 0,04mm

# Fig. 3

Fig. 4

## Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE OS2612846 A
- WO 2008155722 A
- WO 2008155711 A
- WO 2008155710 A
- WO 2008155702 A
- WO 2008155701 A
- WO 2008155699 A
- EP 1225857 A
- WO 0115647 A
- WO 2011120504 A
- DE 102009049450 A
- WO 2008117109 A
- WO 9711659 A
- EP 0826349 A
- WO 9837846 A
- WO 9511653 A
- WO 9511651 A
- WO 9511652 A
- WO 9511654 A
- WO 2004071363 A
- WO 0189439 A
- DE 19529348 A1
- WO 9934843 A1
- WO 2004037903 A2
- EP 0644207 A
- US 7163966 B
- WO 2010115671 A
- WO 2004037903 A
- US 4286082 A
- DE 2706135 A
- US 4076663 A
- DE 3503458 A
- DE 4020780
- DE 4244548 A
- DE 4333056 A
- DE 4418818 A
- DE 2706135A1 A
- DE 4020780 C1
- US 4340706 A
- DE 3713601 A
- DE 2840010 A
- WO 9605234 A

**In der Beschreibung aufgeführte Nicht-Patentliteratur**

- Modern Superabsorbent Polymer Technology. Wiley-VCH, 1998